(19) Europäisches Patentamt
European Patent Office
Office européen des brevets

(11) Veröffentlichungsnummer: **0 444 560 A1**

(12) # EUROPÄISCHE PATENTANMELDUNG

(21) Anmeldenummer: **91102711.8**

(22) Anmeldetag: **25.02.91**

(51) Int. Cl.[5]: **C12P 21/08**, C12N 5/20, C07K 3/28, A61K 39/395

(30) Priorität: **28.02.90 DE 4006269**

(43) Veröffentlichungstag der Anmeldung: **04.09.91 Patentblatt 91/36**

(84) Benannte Vertragsstaaten: **AT BE CH DE DK ES FR GB GR IT LI LU NL SE**

(71) Anmelder: **Max-Planck-Gesellschaft zur Förderung der Wissenschaften e.V.**
**Bunsenstrasse 10**
**W-3400 Göttingen(DE)**

(72) Erfinder: **Pfizenmaier, Klaus, Dr.**
**Lindenallee 7**
**W-3400 Göttingen(DE)**
Erfinder: **Scheurich, Peter, Dr.**
**Uhlandstrasse 18 a**
**W-3406 Bovenden(DE)**
Erfinder: **Thoma, Bettina, Dr.**
**Rastenburgerstrasse 13**
**W-3400 Göttingen(DE)**

(74) Vertreter: **Huber, Bernhard, Dipl.-Chem. et al**
**Möhlstrasse 22 Postfach 860 820**
**W-8000 München 86(DE)**

(54) **Antikörper gegen den Tumor-Nekrosefaktor-Rezeptor.**

(57) Die Erfindung betrifft einen monoklonalen Antikörper gegen ein Membranprotein auf menschlichen Zellen, der bei Bindung an menschliche Zellen, die einen TNF-Rezeptor besitzen, die bekannten Wirkungen von TNF$\alpha$ oder/und TNF$\beta$ zumindest weitgehend neutralisiert, sowie modifizierte Antikörper, Verfahren zur Gewinnung und die Verwendung als Arzneimittel.

EP 0 444 560 A1

Der Tumor-Nekrose-Faktor α (TNFα, Cachectin) und der Tumor-Nekrose-Faktor β (TNFβ, Lymphotoxin) gehören zur Familie der Cytokine, einer Gruppe von Polypeptid-Mediatoren, die Signale von einer Zelle zur anderen übertragen. Die Cytokine sind wesentlich an der molekularen Verständigungsbasis von Entzündungs- und Immunreaktionen des Körpers beteiligt, die ein komplexes Netzwerk von Signalen bildet, welche die Abwehrreaktionen des Körpers aufeinander abstimmen.

Der Tumor-Nekrose-Faktor wird bereits zu einem sehr frühen Zeitpunkt einer Entzündungs- oder Immunreaktion ausgeschüttet und regt dann seinerseits die Immunzellen zur Bildung vieler weiterer Polypeptide an.

Menschlicher TNFα wird als Protein mit 157 A.S. Länge sekretiert, das eine N-terminale Propeptidsequenz von 76 A.S. enthält, so daß reiner TNFα ein relatives Molekulargewicht von 17 kD besitzt (Beutler und Cerami, Nature 320 (1986), 584-588). Menschlicher TNFβ unterscheidet sich strukturell von TNFα durch das Vorhandensein von starker Glykosylierung und das Fehlen einer internen Disulfidbrücke, besitzt jedoch eine 28%ige Homologie auf Aminosäure-Ebene zu TNFα. Obwohl die Synthese beider Cytokine aufgrund relativ unterschiedlicher Stimuli erfolgt, besitzen sie ein stark übereinstimmendes Spektrum an biologischen Aktivitäten und binden an einen gemeinsamen Rezeptor (Aggarwal et al., Nature 318 (1985), 665-667). Die für TNFα und TNFβ kodierenden Gene liegen sowohl bei Menschen (Nedwin et al., Nucleic Acids Res. 13 (1985), 6361-6373) als auch bei der Maus (Nedospasov et al., Nucleic Acids Res. 14 (1986), 7713-7725) in sehr enger Nachbarschaft auf einem Chromosom.

TNF-Rezeptoren werden auf allen somatischen Zellen des Körpers, mit Ausnahme der Erythrozyten, gefunden. Die Rezeptorzahl reicht von einigen 100 Kopien auf bestimmten Zellen bis über 20.000 auf anderen. Die Anzahl der Rezeptoren steht jedoch in den meisten Fällen in keinem Zusammenhang mit der Empfindlichkeit der Zellen. In manchen Fällen, z.B. bei T-Lymphozyten, ist der Rezeptor in ruhenden Zellen nicht (oder in einer inaktiven Form) vorhanden, kann jedoch durch primäre Aktivierung induziert werden (Scheurich et al., J. Immunol. 138 (1987), 1786-1790). Der Rezeptor besitzt eine hohe Affinität für seinen Liganden mit einer Dissoziationskonstante von ungefähr $1x10^{10}M^{-1}$ (Scheurich et al., Int. J. Cancer (1986) 38, 127-133). Nach Gelfiltrationsexperimenten scheint der TNF-Rezeptor ein Komplex aus mehreren verschiedenen Proteinen mit einem relativen Molekulargewicht von ca. 300 kD zu sein (Smith et al., J. Biol. Chem. 261 (1986), 14871-14874). In Zellen unterschiedlichen Ursprungs scheint die dominierende ligandbindende Untereinheit des Rezeptors ein MG von ca. 80 bis 90 kD zu besitzen, während in Zellen epithelialen Ursprungs zusätzlich eine immunologisch damit nicht kreuzreagierende Untereinheiten von etwa 60 kD vorliegt (Hohmann et al., J. Biol. Chem. 264 (1989), 14927-14934). Es scheinen daher offenbar mindestens zwei unterschiedliche Typen von TNF-Rezeptoren zu existieren. Die genaue Natur der Signalübermittlung nach Bindung von TNF an den Rezeptor wurde bisher noch nicht aufgeklärt, es wurde jedoch gefunden, daß die Antwort auf dieses Signal in verschiedenen Geweben stark unterschiedlich ist.

Eine Verabreichung von TNF kann den Organismus vor bakteriellen Infektionen und hohen Strahlendosen schützen. Eine Behandlung mit TNF kann auch das Absterben von Tumoren bewirken, wobei Blutungen in den Tumoren ausgelöst werden, die anschließend austrocknen und absterben. Weiterhin ist TNF offenbar entscheidend an der Vermittlung der Reaktion des Körpers auf eine bakterielle Infektion, z.B. Fieber beteiligt und hilft dem Körper, die Infektion zu überwinden. Somit besitzt TNF - alleine und in Verbindung mit anderen Cytokinen - ein sehr bedeutsames therapeutisches Potential.

Andererseits kann TNF auch eine Reihe pathologischer Zustände hervorrufen, z.B. Kachexie, Gewebeschäden, gramnegativen septischen Schock durch bakterielle Endotoxine und Gehirnentzündung bei Malaria. Weiterhin wurde ein cytotoxischer Effekt von TNF bei Zellen gefunden, die mit HIV infiziert waren (Matsuyama et al., J. Virol. 63 (1989), 2504-2509). Man findet sogar, daß auf molarer Basis die Toxizität von TNF bei manchen Arten etwa 100.000fach höher ist als die von Cyanid (Beutler und Cerami, Nature 320 (1986), 584-588).

Daher ist offenbar die Konzentration im Organismus für die Wirkung von TNF entscheidend. Wie bei vielen anderen im Körper gebildeten Faktoren scheint es auch beim TNF einen schmalen Grad zwischen Nutzen und Schaden zu geben: ein Wirkstoff kann extrem toxisch werden, wenn er in großen Mengen oder am falschen Ort freigesetzt wird. Diese hohe Toxizität von TNF bringt wiederum große Probleme bei seinem möglichen Einsatz als Therapeutikum, z.B. bei Tumorerkrankungen, mit sich. Somit war es Aufgabe der vorliegenden Erfindung, ein Mittel zu entwickeln, das bei Vorliegen von unphysiologisch hohen TNF-Spiegeln im Körper, entweder hervorgerufen durch eine überhöhte endogene Produktion von TNF oder durch eine Behandlung mit TNF - insbesondere wenn eine lokale Anwendung mit hohen Dosen erforderlich ist - die pathologischen Symptome von TNF, insbesondere das Auftreten von Gewebeschäden und Schockzuständen, verringern oder beseitigen kann.

Die erfindungsgemäße Aufgabe wird durch einen monoklonalen Antikörper gegen ein Membranprotein auf menschlichen Zellen gelöst, der bei Bindung an menschliche Zellen, die einen TNF-Rezeptor besitzen, die bekannten Wirkungen von TNF$\alpha$ oder/und TNF$\beta$ zumindest weitgehend neutralisiert.

Der erfindungsgemäße Antikörper ist gegen ein Oberflächenprotein der menschlichen Zelle mit 60 kD gerichtet, bei dem es sich offenbar um einen Bestandteil des TNF-Rezeptorkomplexes handelt. Kompetitionsstudien zeigen, daß der erfindungsgemäße Antikörper in verschiedenen Zellinien unterschiedlich stark mit der Bindung von TNF an seinen Rezeptor interferiert (Tabelle 1). Überraschenderweise ergibt sich jedoch durch Bindung des erfindungsgemäßen Antikörpers bei allen untersuchten Zellinien verschiedenen Gewebeursprungs (lymphoide, myeloide, epitheliale und Fibroblasten-Zellen) unabhängig vom Ausmaß der Kompetition mit der TNF-Bindung eine vollständige Hemmung der zellulären Reaktion auf TNF. Aus diesem Befund läßt sich annehmen, daß der erfindungsgemäße Antikörper möglicherweise ein für die Signalvermittlung sehr unterschiedlicher TNF-Antworten essentielles Rezeptor-Protein erkennt und funktionell blockiert.

Vorzugsweise kann ein erfindungsgemäßer Antikörper die beobachtete cytostatische/cytotoxische Wirkung von TNF auf menschliche Zellen in Gewebekultur (Pfizenmaier et al., Blut, 55, 1-10 (1987a), sogar vollständig neutralisieren. Die antagonistische Wirksamkeit des erfindungsgemäßen Antikörpers wurde an drei verschiedenen menschlichen Zellinien untersucht. In allen Systemen wurde der cytotoxische bzw. cytostatische Effekt von TNF$\alpha$ zusätzlich durch die Anwesenheit von Interferon-Gamma oder Proteinsynthese-Hemmern (z.B. Cycloheximid) verstärkt, wobei IFN-Gamma bzw. Proteinsynthese-Hemmer unter den eingesetzten Bedingungen für sich alleine keinen Einfluß auf die gemessenen zellulären Reaktionen haben. Es wurde festgestellt, daß eine Bindung des erfindungsgemäßen Antikörpers die cytotoxische/cytostatische Wirkung von TNF auf alle drei untersuchten menschlichen Zellinien vollständig neutralisiert. Auf ähnliche Weise neutralisiert ein erfindungsgemäßer Antikörper auch die Wirkung von TNF$\beta$ und murinem TNF$\alpha$, welches auf menschlichen Zellen ebenso wirksam ist.

Weiterhin wirkt ein erfindungsgemäßer Antikörper auch als Antagonist der TNF-induzierten Expression von HLA-Genen. Die TNF-induzierte Modulation von HLA-Genen wurde in einer menschlichen Zellinie myeloischen Ursprungs (K562) und einer epithelialen Karzinomzellinie (Colo 205) untersucht. In beiden Systemen wurde zusätzlich eine Co-Stimulation von TNF$\alpha$ zusammen mit Interferon-Gamma durchgeführt, um die jeweilige Reaktion auszulösen (Pfizenmaier et al., J. Immunol. 138, 975-980 (1987b). In Anwesenheit eines erfindungsgemäßen Antikörpers kann es bei beiden Systemen zur vollständigen Hemmung der TNF-Wirkung kommen.

Schließlich wirkt ein erfindungsgemäßer Antikörper auch als Antagonist der durch TNF vermittelten Immunstimulation. So kann die Stimulation der Interleukin-2-Rezeptor-Expression auf T-Lymphozyten und auf sogenannten natürlichen Killerzellen (NK-Zellen) (Scheurich et al., (1987) supra) durch Bindung eines erfindungsgemäßen Antikörpers vollständig inhibiert werden.

Ein besonders bevorzugter Gegenstand der vorliegenden Erfindung ist ein spezieller Antikörper H398 mit der Fähigkeit, die bekannten TNF-Wirkungen vollständig zu neutralisieren, bei dem es sich um ein Immunglobulin vom Isotyp IgG2a handelt und der von der nach Budapester Vertrag hinterlegten Hybridom-Zellinie ECACC 90021617 (bzw. H398.6.1) exprimiert wird.

Weiterhin ein Gegenstand der vorliegenden Erfindung ist auch ein Protein mit Antikörper-Eigenschaften, das eine gleiche Bindungsspezifität wie der erfindungsgemäße monoklonale Antikörper besitzt. Besonders bevorzugt in diesem Sinne ist ein Protein mit Antikörper-Eigenschaften, das im wesentlichen die gleiche Antigen-Bindungsstelle wie der erfindungsgemäße monoklonale Antikörper H398 besitzt.

Unter dem Begriff "Protein mit Antikörper-Eigenschaften" sind mittels rekombinanter DNA-Technologie modifizierte Antikörper zu verstehen. Um die Wirkung von TNF zumindest weitgehend zu neutralisieren, ist es erforderlich, daß solche modifizierten Proteine die gleiche Bindungsspezifität wie der erfindungsgemäße monoklonale Antikörper besitzen, d.h. sie müssen an ein 60 kD Membranprotein auf menschlichen Zellen binden, das ein Bestandteil des TNF-Rezeptorkomplexes ist, und sie müssen in der Lage sein, in einem therapeutisch vertretbaren Konzentrationsbereich die bekannten Wirkungen von TNF$\alpha$ oder/und TNF$\beta$ zumindest weitgehend zu neutralisieren. Um diese Kriterien zu erfüllen, muß das modifizierte Protein mit Antikörper-Eigenschaften eine im wesentlichen gleiche Antigen-Bindungsstelle wie der erfindungsgemäße monoklonale Antikörper aufweisen. Dem Fachmann auf dem Gebiet der Immunologie ist bekannt, daß die Bindungsspezifität durch ganz bestimmte Bereiche des Antikörpers, nämlich die hypervariablen Regionen, charakterisiert ist. Somit ist es bei einer Abänderung eines erfindungsgemäßen Antikörpers zur Herstellung eines modifizierten Proteins mit Antikörper-Eigenschaften wesentlich, daß keine, die Bindungsspezifität verändernden Modifikationen in den hypervariablen Regionen des Antikörpers erfolgen. Hingegen ist es möglich, andere Regionen des Antikörpers (konstante Region oder/und die nicht hypervariablen Teile der variablen Region) zu verändern, um dem modifizierten Protein gewisse gewünschte Eigenschaften zu verleihen, ohne daß sich die Bindungs-Charakteristika des Antikörpers wesentlich ändern. Demnach beinhaltet die Erfindung auch modifizierte Proteine wie Antikörper-Teilfragmente (z.B. $F(ab)_2$), einkettige Antikörper oder chimärisierte, insbesondere humanisierte Antikörper (Ersetzen der nicht an der Bindung beteiligten Antikörperbereiche durch Sequenzen eines menschlichen Antikörpers). Besonders bevorzugt sind in diesem Sinne Proteine mit Antikörper-Eigenschaft, welche im wesentlichen den gleichen Antigen-Bindungsbereich wie der Antikörper H398 besitzen.

Weiterhin ein Gegenstand der vorliegenden Erfindung ist ein Verfahren zur Gewinnung eines erfindungsgemäßen monoklonalen Antikörpers, bei dem man auf übliche Weise Myelomzellen mit Milzzellen oder T-Lymphozyten einer Maus fusioniert, die zuvor mit Affinitäts-gereinigtem TNF-Rezeptor-Material aus menschlichen P60 Antigen-positiven Zellen, z.B. HL-60 (ATCC No. CCL 240) hyperimmunisiert wurde.

Zur Reinigung des TNF-Rezeptors werden zunächst Zellmembranen präpariert, anschließend solubilisiert und über eine TNF-Affinitätssäule gereinigt. Vorzugsweise erfolgt die Präparation der Zellmembranen durch Homogenisation in einem Glas/Glas-Homogenisator, wobei gegebenenfalls eine Vorbehandlung in einem hypotonen Medium erfolgen soll. Dann erfolgt zunächst eine Abtrennung der Zellkerne und der noch intakten Zellen, anschließend erfolgt eine Pelletierung der Zellmembranen durch Zentrifugation. Die Solubilisierung des TNF-Rezeptor-Proteins erfolgt erfindungsgemäß mit einem Puffer, der ein nicht-ionisches Detergens, vorzugsweise etwa 1 % Triton X-100 enthält. Der Solubilisierungs-Puffer kann zusätzlich auch Leupeptin, Aprotinin und den Protease-Hemmstoff Di-isopropylfluorophosphat (DFP) enthalten. Die TNF-Affinitätssäule wird dabei durch Kopplung von TNF, vorzugsweise rekombinantem humanem TNFα an ein geeignetes Trägermaterial, vorzugsweise Affigel -15, hergestellt. Die Elution des gebundenen Rezeptors erfolgt im sauren Bereich bei pH 3,0. Anschließend kann noch ein weiterer Reinigungsschritt, eine Acetonfällung, erfolgen. Dazu wird die Probe mit ungefähr 3 Teilen Aceton versetzt und eine ausreichende Zeit bei einer ausreichend niedrigen Temperatur, vorzugsweise 14 Stunden bei -80°C, aufbewahrt. Der entstandene Niederschlag, der das Rezeptor-Protein enthält, wird abzentrifugiert und in einem geeigneten Puffer wieder aufgenommen.

Die Hyperimmunisierung einer Maus mit dem so gewonnenen Rezeptormaterial geschieht vorzugsweise in drei Schritten. Im ersten Schritt (Tag 1) wird 1 µg Rezeptorprotein mit komplettem Freund'schen Adjuvans intraperitoneal verabreicht, im zweiten Schritt (Tag 40) wird ebenfalls 1 µg Rezeptorprotein, versetzt mit inkomplettem Freund'schen Adjuvans, intraperitoneal verabreicht und im dritten Schritt (Tag 55) werden 2 µg Rezeptorprotein ohne Adjuvans verabreicht. Die Entnahme der Milz erfolgt vorzugsweise am 58. Tag unter sterilen Bedingungen. Die Herstellung von Hybridom-Zellinien erfolgt nach den üblichen Verfahren. Die Immunisierung einer Maus zur Erzeugung eines genügend hohen Antikörperspiegels gegen den TNF-Rezeptor ist jedoch auch durch andere geeignete Immunisierungsprotokolle möglich, die dem Fachmann bekannt sind.

Die Fusion der Milzzellen einer, mit dem TNF-Rezeptor-Protein hyperimmunisierten Maus, kann auf übliche Weise erfolgen. Die resultierenden Hybridomklone wurden auf eine Modulation der TNF-Bindungsfähigkeit an der TNF-Rezeptor-positiven menschlichen Leukämiezellinie HL-60 (ATCC Nr. CCL 240) getestet.

Aus 413 untersuchten Hybridom-Klonen konnte auf diese Weise eine Hybridom-Zellinie gewonnen werden, welche einen erfindungsgemäßen Antikörper sekretiert.

Weiterhin ein Gegenstand der Erfindung ist ein Arzneimittel, das einen erfindungsgemäßen monoklonalen Antikörper oder/und ein erfindungsgemäßes Protein mit Antikörper-Eigenschaften als Wirkstoff enthält, gegebenenfalls mit üblichen Träger-, Hilfs- oder/und Füllstoffen.

Für die biologische Wirkung von TNFα und TNFβ ist die Bindung an spezifische Zellmembran-Rezeptoren mit hoher Affinität eine Voraussetzung (Pfizenmaier et al. (1987a) supra). Der erfindungsgemäße Antikörper kann daher diagnostisch eingesetzt werden, um zelluläre TNF-Rezeptoren nachzuweisen und potientielle TNF-reaktive Zellen in verschiedenen Geweben/Organen zu identifizieren. Darüberhinaus eignet sich der obengenannte Antikörper zum Nachweis sezernierter Rezeptorfragmente im Serum und anderen Körperflüssigkeiten.

Die größere Bedeutung von erfindungsgemäßen Antikörpern liegt in ihrer Eigenschaft, bekannte biologische Wirkungen von TNFα und TNFβ mindestens weitgehend zu inhibieren. Für eine TNF neutralisierende Wirkung in vitro ist es ausreichend, wenn der Antikörper gemeinsam mit dem Cytokin verabreicht wird; eine Vorinkubation mit anti-Rezeptor-Antikörper ist in vitro nicht erforderlich.

Aufgrund dieser ausgeprägten antagonistischen Eigenschaften in verschiedenen in vitro Experimentalmodellen kommt einem derartigen Antikörper eine sehr große potentielle therapeutische Bedeutung zu. Ein erfindungsgemäßer Antikörper könnte bei all den Erkrankungen Anwendung finden, welche mit erhöhten, pathologischen TNFα/TNFβ-Spiegeln im Serum und/oder anderen Körperflüssigkeiten einhergehen und bei denen TNFα/TNFβ als pathogenes Prinzip erkannt wurde bzw. vermutet wird (Old, Nature 326 (1987), 330-331; Warren et al., Mod. Pathol. 1 (1988), 242-247; Beutler & Cerami, Ann. Rev. Biochem. 57 (1989), 505-518; Grau et al., Immunol. Rev. 112, 49-70 (1989)). Hierzu gehören Autoimmunerkrankungen wie z.B. die rheumatoide Arthritis und Krebserkrankungen, die mit einer deregulierten TNFα/TNFβ-Produktion der malignen Zelle selbst (z.B. Myelom) bzw. des Immunsystems (z.B. $CD4^+$-Zellen bei der chronischen B-lymphatischen Leukämie; B-CLL) einhergehen. Insbesondere ist an einen therapeutischen Einsatz eines erfindungsgemäßen Antikörpers bei TNF-vermittelten, akut lebensbedrohlichen Krankheitsverläufen zu denken, so z.B. beim septischen Schock durch bakterielle Infektionen (Meningokokkensepsis), sowie bei zerebraler Malaria. In diesen Fällen ist zu erwarten, daß schon nach kurzzeitiger Behandlung mit den Antikörpern der Krankheitsverlauf günstig beeinflußt werden kann. Doch auch bei chronischen Erkrankungen wäre eine Therapie mit einem erfindungsgemäßen Antikörper, bzw. dem modifizierten Antikörper (z.B. durch Austausch der konstanten Regionen der Maus durch konstante Regionen des Menschen) möglich.

Ein weiteres potentielles Einsatzgebiet des Antikörpers liegt in der Mitbehandlung der AIDS-Erkrankung. Da die Vermehrung von HIV, dem Erreger von AIDS, durch TNF stimuliert wird und TNF somit als einer der Progressionsfaktoren vom latenten zum akuten Krankheitsbild angesehen wird (Matsuyama et al., J. Virol. (1989), 2504-2509) könnte der Antikörper H398 als antiviral wirksames Prinzip bei solchen AIDS-Patienten eingesetzt werden, bei denen entweder schon konstitutiv erhöhte TNF-Spiegel festgestellt werden (Lahdevirta et al., Am. J. Med. 85 (1989), 289-291) oder bei denen durch exogene immunstimulierende Maßnahmen (z.B. spezifisch aktive Immunisierung) eine TNF-Produktion induziert werden könnte (Matsuyama et al., (1989) supra). Da der erfindungsgemäße Antikörper H398 in vitro auch inhibitorisch auf die TNF-stimulierte Aktivität des HIV-Promotors wirkt, der die Transkription der retroviralen Gene kontrolliert, könnte der Einsatz eines erfindungsgemäßen Antikörpers einer TNF-induzierten Virustranskription und damit einer Vermehrung infektiöser Viren entgegenwirken.

Die Dosierung des erfindungsgemäßen Antikörpers zum Antagonisieren unerwünschter Nebenwirkungen von TNF ist natürlich vom Einzelfall abhängig, insbesondere bei einer Verabreichung von TNF, beträgt aber vorzugsweise zwischen 0,1 und 100 mg pro Person.

Die folgenden Beispiele sollen zusammen mit den Abbildungen 1 bis 3 die Erfindung näher verdeutlichen.

Es zeigen:

Abb. 1 die spezifische Reaktion des Antikörpers H398: er präzipitiert ein jodmarkiertes Membranprotein mit dem MW 60 kDa aus HL-60 Zellen,

Abb. 2 die Neutralisierung der durch TNF hervorgerufenen cytotoxischen bzw. cytostatischen Wirkung durch den Antikörper H398,

Abb. 3 die Neutralisierung der durch TNF induzierten HLA-Genexpression durch den Antikörper H398.

**Beispiel 1**

Reinigung des TNF-Rezeptorproteins

1.1 Präparation von Zellmembranen

Puffer A: 125 mM Sucrose
7,5 mM Tris/HCl pH 7,4
2 mM EDTA

Puffer B: 100 mM Tris/HCl pH 7,4
10 mM $MgCl_2$
10 mM $CaCl_2$
2 % Fötales Kälberserum
(Seramed, Biochrom KG, Berlin)

Jeweils am Präparationstag wurden die Puffer A und B mit 1 mM Phenylmethylsulfonyfluorid (PMSF) versetzt. Die frisch geernteten Zellen wurden vor der Homogenisation in einem Glas/Glas-Homogenisator bei einer Zelldichte von $1 \cdot 10^8$/ml in Puffer A für 30 Minuten inkubiert. Diese Vorbehandlung in hypotonem Medium erleichterte die anschließende Homogenisation mit 30 Schüben bei 1000 Upm. Das Homogenat wurde mit dem gleichen Volumen Puffer A verdünnt und bei 50 x g für 10 Minuten zentrifugiert, um Kerne sowie noch intakte Zellen abzutrennen. Das Sediment wurde einmal mit dem gleichen Volumen Puffer A gewaschen und die vereinigten Überstände bei 12000 x g für 30 Minuten zentrifugiert. Die pelletierten Membranen wurden anschließend zweimal mit Puffer B gewaschen. Alle Präparationsschritte wurden bei 4° C durchgeführt.

1.2 Solubilisierung des TNF-Rezeptors

Solubilisierungspuffer: 20 mM Tris/HCl pH 7,4
2 mM $MgCl_2$
2 mM $CaCl_2$

1 % Triton X-100
10 $\mu$g/ml Leupeptin
1000 KIE/ml Aprotinin
0,5 $\mu$l/ml DFP

Der Proteasehemmstoff DFP (Diisopropyl-fluorophosphat (Sigma) wurde jeweils frisch zum Puffer gegeben. Das Membranpellet wurde in eiskaltem Solubilisierungspuffer mit 1 ml pro 5 x $10^8$ eingesetzter Zellen suspendiert und mit 10 Schüben in einem Glas/Glas-Homogenisator bei 1000 Upm homogenisiert. Nach einstündiger Inkubation auf Eis wurde der Ansatz für 30 Minuten bei 100.000 x g zentrifugiert und nur der klare Überstand weiter verwendet.

1.3 Herstellung der TNF-Affinitätssäule

Zur Herstellung der TNF-Affinitätssäule wurden 5 mg gereinigtes, rekombinantes, humanes TNF$\alpha$ an 1 ml Affigel 15 gekoppelt, entsprechend der Vorschrift des Herstellers (Biorad). Zuvor wurde das TNF 24 Stunden in einem geeigneten Dialyseschlauch gegen den Kopplungspuffer (0,1 M MOPS/HCl pH 7,5) dialysiert. Das Affigel wurde dreimal mit bidestilliertem Wasser gewaschen und mit der dialysierten TNF$\alpha$-Lösung 4 Stunden kräftig geschüttelt. Die nicht umgesetzten reaktiven Gruppen des Affigels wurden danach durch Zugabe von 1 M Tris/HCl pH 7,4 (Endkonzentration 50 mM) für eine Stunde abgesättigt. Anschließend wurde das TNF-Affigel zweimal mit Waschpuffer pH 7,4 (siehe 1.4) gewaschen und in eine FPLC-Säule (HR 5/5, Fa. Pharmacia, Freiburg) gefüllt. Vor der ersten Verwendung der Säule wurde das gesamte Wasch- und Elutionsprogramm der Affinitätsreinigung mit allen dort verwendeten Puffern einmal durchgeführt. Die gesamte Herstellung der Säule wurde bei 4° C durchgeführt; ebenfalls bei 4° C wurde sie in Waschpuffer pH 7,4, versetzt mit 20 mM Natriumazid, aufbewahrt.

1.4 Affinitätschromatographie

| | |
|---|---|
| Waschpuffer pH 7,4: | 10 mM Tris/HCl pH 7,4 |
| | 150 mM NaCl |
| | 0,1 % Triton X-100 |
| Waschpuffer 3D: | 10 mM Tris/HCl pH 7,4 |
| | 150 mM NaCl |
| | 10 % Glycerin |
| | 1 % Natriumdesoxycholat |
| | 0,1 % SDS |
| | 0,1 % Triton X-100 |
| Waschpuffer pH 8,6: | 10 mM Tris/HCl pH 8,6 |
| | 500 mM KCl |
| | 0,5 % Triton X-100 |
| Waschpuffer pH 4,5: | 50 mM Glycin/HCl pH 4,5 |
| | 150 mM NaCl |
| | 0,1 % Triton X-100 |
| Elutionspuffer pH 3,0: | 150 mM Glycin/HCl pH 3,0 |
| | 0,1 % Triton X-100. |

Die gesamte Affinitätsreinigung von solubilisiertem Material wurde auf einer FPLC-Anlage (Fa. Pharmacia, Freiburg) bei 4° C durchgeführt. Zur Proben- bzw. Pufferaufgabe wurde eine Schleife (Superloop) mit 50 ml Fassungsvermögen verwendet.

Der Membranextrakt von 7•$10^{10}$ Zellen wurde mit einer Flußgeschwindigkeit von 0,2 ml/min auf die TNF-Affinitätssäule aufgegeben, und anschließend wurde die Säule mit einer Flußgeschwindigkeit von 0,5 ml/min mit den Puffern in folgender Reihenfolge (s.u.) gewaschen:

1) 10 ml Waschpuffer pH 7,4
2) 10 ml Waschpuffer 3D
3) 2 ml Waschpuffer pH 7,4
4) 10 ml Waschpuffer pH 8,6
5) 2 ml Waschpuffer pH 7,4
6) 5 ml Waschpuffer pH 4,5

Der Rezeptor wurde durch den sauren Elutionspuffer (pH 3,0) mit einer Flußgeschwindigkeit von 0,1 ml/min von der Säule eluiert. Dabei wurde das Eluat in 10 Fraktionen zu je 1 ml gesammelt und durch 200 $\mu$l eines 1 M Tris/HCl Puffers pH 7,4, der in die Fraktionsröhrchen vorgelegt wurde, sofort neutralisiert. Die Fraktionen wurden bei -20° C aufbewahrt.

1.5 Fällung von Proteinen mit Aceton

Das gesammelte Eluat wurde mit -80° C kaltem Aceton in einem Verhältnis von 1 Teil Probe/3 Teile Aceton versetzt und 14 Stunden bei -80° C aufbewahrt. Nach dieser Zeit wurde der entstandene Niederschlag 45 Minuten bei 12000 x g in einer kühlbaren Zentrifuge bei 0 °C pelletiert. Der Überstand wurde anschließend sofort abgesaugt und das Pellet in 400 µl 20 mM Tris-Puffer, pH 7,4, versetzt mit 0,1 % Triton X-100, wieder aufgelöst. TNF-Bindungsstudien zeigten, daß diese Präparation insgesamt 4 µg aktives Rezeptorprotein enthielt (Gesamtproteingehalt: 80 µg).

2. Immunisierung

Eine 6 Wochen alte weibliche Maus vom Stamm BALB/c wurde mit dem Rezeptormaterial nach folgendem Schema immunisiert:

Tag 1: Intraperitoneale Injektion von 100 µl Rezeptorpräparation (1 µg Rezeptorprotein), versetzt mit komplettem Freund'schen Adjuvans (Behringwerke AG, OREC 20/21) (100 µl),

Tag 40: Intraperitoneale Injektion von 100 µl (1 µg Rezeptorprotein), versetzt mit inkomplettem Freund'schem Adjuvans (Behringwerke AG, ORED 20/21),

Tag 55: Intraperitoneale Injektion von 200 µl (2 µg Rezeptorprotein), ohne Adjuvans

Tag 58: Entnahme der Milz unter sterilen Bedingungen, Herstellung einer Milzzellsuspension zur Zellfusion mit Myelomzellen.

3. Herstellung von Hybridomen

Die Fusion der Milzzellen einer hyperimmunisierten BALB-C-Maus mit NSO-Zellen wurde genau nach Vorschrift von Peters et al. (Monoklonale Antikörper, Springer Verlag, Berlin, Heidelberg, 1985) im Verhältnis 5:1 durchgeführt und nach Standardselektionsbedingungen (HAT-Medium) kultiviert. Insgesamt wurden 413 Hybridklone isoliert und nach Transfer in Mikrotiterplatten auf Reaktion mit TNF-R-positiven Zellen (HL-60) untersucht.

4. Screening-Verfahren zur Identifikation von TNF-R-spezifischen, monoklonalen Antikörpern: Modulation der TNF-Bindungsfähigkeit

$1 \times 10^6$ HL-60 Zellen wurden mit Hybridkulturüberständen (20 % Endkonzentration) für 2 Stunden bei 37 °C inkubiert, der Überstand nach Zentrifugation abgesaugt und die Zellen für weitere 60 Minuten bei 4 °C mit radioaktivem TNF (20 ng/ml) inkubiert. Die spezifische TNF-Bindung solcher Zellen wurde mit der von unbehandelten (NSO-Überstände) Kontrollzellen verglichen. Von den 413 untersuchten Klonen erwies sich einer (H398) nach zweifacher Subklonierung als stabiles IgG produzierendes Hybridom, welches reproduzierbar die TNF-Bindung blockiert.

5. Immunpräzipitation des TNF-Rezeptors p60 mittels Antikörpern H398

$2 \times 10^8$ HL60 Zellen/Gruppe wurde nach der Lactoperoxidase-Methode oberflächenjodiert, gewaschen und mit Triton X-100 (1 %) solubilisiert. Aus dem Triton X-100-Extrakt wurden nach Vorinkubation (Preclearing) mit Prot-A-Sepharose (2 Stunden 0 °) mit Prot-A-Sepharose gekoppeltem mAK H398 bzw. IgG-Kontrollantikörper (Spur C) immunpräzipitiert (16 Stunden, 0 °C), das Präzipitat in Tris Puffer (20 mM) 0,1 % Triton X-100, 1 M NaCl (pH 7,8) dreimal gewaschen, in SDS-Probenpuffer 3' bei 100 °C inkubiert und der Überstand auf einer 7,5 % PAGE unter nicht reduzierten Bedingungen aufgetrennt. Abb. 1 zeigt ein Autoradiogramm nach 64 Stunden Exposition des getrockneten Gels. Man erkennt nur auf der Spur des H398-Immunpräzipitates eine spezifische Bande bei 60 kD. Immunpräzipitiertes p60 besitzt auch nach SDS-Gelelektrophorese im Liganden-blot mit jodmarkiertem TNF spezifische Bindungseigenschaften für TNFα.

**Beispiel 2**

Antagonistische Wirkung des Antikörpers H398 auf den cytotoxischen und cytostatischen Effekt von Tumor-Nekrose-Faktoren

1. H398 als TNF-Antagonist bei der zytotoxischen Wirkung von TNF auf HeLa- und MCF-7-Zellen.

Menschliche HeLa- bzw. MCF-7-Zellen werden in Mikrokulturen ($3 \times 10^4$ Zellen in 0,2 ml Kulturmedium Clicks/RPMI (Kat.Nr. T125); 5 % Fötales Kälberserum (beide Seramed, Biochrom, Berlin, FRG); für 18 Stunden vorkultiviert, dann für 7 Stunden in Gegenwart von 5 µg/ml Cycloheximid mit 1 ng/ml (50 Units) TNF und seriellen Verdünnungen von H398 behandelt. Danach wird die Anzahl überlebender Zellen durch Anfärben mit Kristallviolett quantifiziert. Abbildung 2 (graue Säule) zeigt, daß H398 HeLa-Zellen vor der zytotoxischen TNF-Wirkung konzentrationsabhängig schützt. Vergleichbare Daten wurden mit MCF-7 erhalten (Tabelle 1).

TABELLE 1

Gegenüberstellung der Fähigkeit von H398 mit der Bindung von $^{125}$J-TNF zu kompetieren und seiner antagonistischen Wirkung bei der zellulären TNF-Antwort

| Zellinie | Kompetition der TNF-Bindung % Inhibition[1)] | antagonistische Wirkung % Inhibition der zellulären Reaktion auf TNF[2)] | Testsystem |
|---|---|---|---|
| U937 (Mensch) | 50 | 100 | Wachstumshemmung |
| K562 " | 20 | 100 | HLA-A,B,C Expression |
| Colo 205 (Mensch) | 20 | 100 | HLA-DR Expression |
| HeLa (Mensch) | 88 | >77[3)] | Zytotoxizität |
| MCF7 (Mensch) | 96 | >66[3)] | Zytotoxizität |
| YT (Mensch) | 10 | 100 | IL-2 Rezeptorexpression |
| L929 (Maus) | 2 | <5 | Zytotoxizität |

1) Angegeben ist die Reduktion der spezifischen $^{125}$J-TNF-Bindung (20 ng/ml $^{125}$J-TNF wurden mit 10 $\mu$g/ml H398 gemischt und mit $1x10^6$ Zellen/Ansatz für eine Stunde bei 0°C inkubiert. Die Bestimmung spez. TNF-Bindung wurde wie beschrieben (Scheurich et al. 1987) durchgeführt.

2) Die angegebenen Werte wurden mit H398 Ascitesverdünnung von $10^{-3}$, entsprechend einer Antikörperkonzentration von 10 $\mu$g/ml erreicht.

3) Wegen unspezifischer toxischer Nebenwirkungen niedriger Aszitesverdünnungen (< $10^{-2}$) konnten hier keine höheren Antikörperkonzentrationen eingesetzt und damit vollständige Hemmung der TNF-Antwort erreicht werden.

2. Blockierung der wachstumshemmenden Wirkung von TNF auf U937-Zellen.

Tumor-Nekrose-Faktoren zeigen auf der menschlichen U937-Zellinie eine wachstumsinhibitorische Wirkung im Synergismus mit Interferon-gamma (Pfizenmaier et al., Blut 55 (1978), 1-10). Die Zellen ($5x10^3$ in 0,2 ml Kulturmedium) wurden für 48 Stunden in Gegenwart von 10 ng/ml Interferon-gamma, 1 ng/ml TNF und seriellen Verdünnungen an H398 kultiviert. Die Proliferationskapazität der U937-Zellen wurden durch einen sechsstündigen $^3$H-Thymidinpuls für 6 Stunden bestimmt. In Anwesenheit von H398 (10 $\mu$g/ml) kommt es zur vollständigen Inhibition der durch TNF vermittelten Zytostase (Tabelle 1 und Abbildung 2, schwarze Säule). In ähnlicher Weise schützt H398 auch vor TNF$\beta$ (Lymphotoxin) und auch vor murinem TNF, welches auch auf menschlichen Zellen wirksam ist.

3. H398 zeigt keine Wirkung auf Mauszellen.

H398 ist speziesspezifisch und erkennt TNF-Rezeptoren auf Humanzellen, nicht aber auf Mauszellen (vgl. auch Tabelle 1). Abb. 2 (weiße Säulen) zeigt, daß H398 auf Mauszellen keinerlei protektive Wirkung für humanen TNF besitzt. Der Experimentalansatz entspricht dem Standardtestsystem zur Bestimmung von TNF (24 Stunden Kultur von murinen L929-Zellen in der Gegenwart von 1 $\mu$g/ml Actinomycin D, 1 ng/ml TNF) in An- bzw. Abwesenheit von H398-Antikörper (10 $\mu$g/ml).

**Beispiel 3**

Antagonistische Wirkung des Antikörpers H398 auf die durch TNF induzierte Expression von HLA-Genen

Die TNF-induzierte Modulation von HLA-Genen wurde in einer Zellinie myeloischen Ursprungs (K562) und einer epithelialen Karzinomzellinie (Colo 205) untersucht. In beiden Systemen ist eine Kostimulation mit Interferongamma notwendig, um die jeweilige Reaktion auszulösen (Pfizenmaier et al., J. Immunol. 138 (1987), 975-980). In Anwesenheit von H398 kommt es in beiden Systemen zur vollständigen Inhibition der TNF-Antwort (vgl. auch Tabelle 1).

1. H398 als Antagonist der TNF-verstärkten HLA-A,B,C Antigenexpression auf K562.

K562-Zellen wurden für 24 Stunden in Gegenwart von 10 ng/ml Interferon-gamma, 3 ng/ml TNF und seriellen Verdünnungen von H398 kultiviert ($5x10^5$ Zellen in 2 ml Kulturmedium). Danach wurden die Zellen geerntet und die HLA-Antigenexpression wurde mit Hilfe HLA-spezifischer monoklonaler Antikörper durch indirekte Immunfluoreszenzanalyse im Durchflußzytofluorographen quantifiziert. Die Ergebnisse sind in Abbildung 3 (graue Säule) dargestellt und zeigen, daß bei 10 $\mu$g/ml H398 ($10^{-3}$ Verdünnung einer Ascites mit 10 mg/ml H398 monoklonalem Antikörper) die TNF-induzierte Verstärkung der HLA-A,B,C-Antigenexpression vollständig neutralisiert wird. Eine halbmaximale Inhibition wird mit etwa 1 $\mu$g/ml Antikörper erreicht.

2. Antagonistische Wirkung von H398 der TNF-verstärkten HLA-DR Antigenexpression auf Colo 205-Zellen.

Der Experimentalansatz ist ähnlich dem oben beschrieben (jedoch 48 Stunden Kulturdauer, 0,02 ng/ml Interferon-gamma, 1 ng/ml TNF). Die Daten in Abbildung 3 (schwarze Säulen) zeigen ebenfalls eine vollständige Aufhebung der TNF-induzierten Verstärkung der HLA-DR-Genexpression bei 10 $\mu$g/ml H398; die halbmaximale Hemmung wird hier mit etwa 2 $\mu$g/ml H398 erreicht.

**Beispiel 4**

Antagonistische Wirkung des Antikörpers H398 auf die von TNF vermittelte Immunstimulation.

Eine bedeutende immunregulatorische Funktion von TNF ist die Stimulation der Interleukin-2 Rezeptorexpression auf T-Lymphozyten und auf sogenannten Natürlichen Killerzellen (NK-Zellen) (Scheurich et al., J. Immunol. 138 (1987), 1786-1790). Auch in diesem System besitzt H398 antagonistische Wirkung. Es wurden $5x10^5$ Zellen der NK-Zellinie YT in 2 ml Kulturmedium für 24 Stunden in Gegenwart von 1 und 10 ng/ml TNF sowie seriellen Verdünnungen von H398 kultiviert. Die Auswertung erfolgte zytofluorographisch mit Hilfe von anti-Interleukin-2 Rezeptor Antikörpern (anti Tac). Die in Tabelle 1 dargestellten Ergebnisse belegen die komplette Inhibition auch dieser TNF-Antwort durch H398.

**Patentansprüche**

1. Monoklonaler Antikörper gegen ein Membranprotein auf menschlichen Zellen, **dadurch gekennzeichnet,** daß er bei Bindung an menschliche Zellen, die einen TNF-Rezeptor besitzen, die bekannten Wirkungen von TNF$\alpha$ oder/und TNF$\beta$ zumindest weitgehend neutralisiert.

2. Monoklonaler Antikörper nach Anspruch 1, **dadurch gekennzeichnet,** daß er die durch TNF $\alpha$ oder/und TNF $\beta$ stimulierte cytotoxische und cytostatische Wirkung zumindest weitgehend neutralisiert.

3. Monoklonaler Antikörper nach Anspruch 1, **dadurch gekennzeichnet,** daß er die durch TNF $\alpha$

oder/und TNF β stimulierte Expression von HLA-Genen zumindest weitgehend neutralisiert.

4. Monoklonaler Antikörper nach Anspruch 1, **dadurch gekennzeichnet,** daß er die durch TNF α oder/und TNF β vermittelte Immunstimulation von T-Lymphozyten und Natürlichen Killerzellen zumindest weitgehend neutralisiert.

5. Monoklonaler Antikörper H398, der von der Hybridomzellinie ECACC 90021617 exprimiert wird.

6. Protein mit Antikörpereigenschaften, **dadurch gekennzeichnet,** daß es eine gleiche Bindungsspezifität wie ein monoklonaler Antikörper nach einem der Ansprüche 1 bis 5 besitzt.

7. Protein mit Antikörpereigenschaften nach Anspruch 6, **dadurch gekennzeichnet,** daß es im wesentlichen di gleiche Antigen-Bindungsstelle wie ein monoklonale Antikörper nach Anspruch 5 besitzt.

8. Hybridomzellinie ECACC 90021617.

9. Verfahren zur Gewinnung eines monoklonalen Antikörpers nach einem der Ansprüche 1 bis 5, **dadurch gekennzeichnet,** daß man auf übliche Weise Myelomzellen mit Milz- oder B-Zellen einer Maus fusioniert, die zuvor mit Affinitäts-gereinigtem TNF-Rezeptormaterial aus menschlichen Zellen hyperimmunisiert wurde.

10. Verfahren nach Anspruch 9, **dadurch gekennzeichnet,** daß man zur Affinitätsreinigung von TNF-Rezeptormaterial zunächst menschliche Zellen homogenisiert, die Membranen aus dem Homogenat abtrennt und dann mit einem Solubilisierungspuffer behandelt, der ein nicht-ionisches Detergens enthält.

11. Verfahren nach Anspruch 10, **dadurch gekennzeichnet,** daß man als nichtionisches Detergens Triton X-100 verwendet.

12. Verfahren nach einem der Ansprüche 9 bis 11, **dadurch gekennzeichnet,** daß man das TNF-Rezeptormaterial über eine TNF-Affinitätssäule reinigt, auf der sich immobilisierter TNF befindet.

13. Verfahren nach Anspruch 12, **dadurch gekennzeichnet,** daß man rekombinanten humanen TNFα verwendet.

14. Verfahren nach einem der Ansprüche 9 bis 13, **dadurch gekennzeichnet,** daß man das TNF-Rezeptormaterial nach der Affinitätschromatographie noch durch eine Acetonfällung reinigt.

15. Arzneimittel, **gekennzeichnet durch** einen monoklonalen Antikörper nach einem der Ansprüche 1 bis 5 oder/und ein Protein mit Antikörpereigenschaften nach Anspruch 6 oder 7 als Wirkstoff, gegebenenfalls mit üblichen Träger-, Hilfs- oder/und Füllstoffen.

16. Arzneimittel nach Anspruch 15, **dadurch gekennzeichnet,** daß es in einer Dosis von 0,1 bis 100 mg Wirkstoff pro Person verabreicht wird.

17. Verwendung eines monoklonalen Antikörpers nach einem der Ansprüche 1 bis 5 oder/und eines Proteins mit Antikörpereigenschaften nach Anspruch 6 oder 7 zur Behandlung von Krebs, Entzündungen, Erkrankungen des Immunsystems, Schockzuständen und AIDS.

# Abb.1

H398 spezifische Immunpräparation von p60

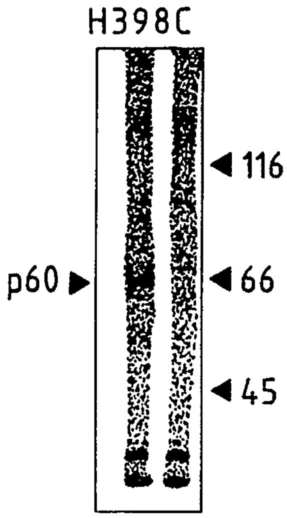

Abb. 2

Anti-TNF-Rezeptor-Antikörper H398:
TNF-Antagonist nur auf menschlichen Zellen

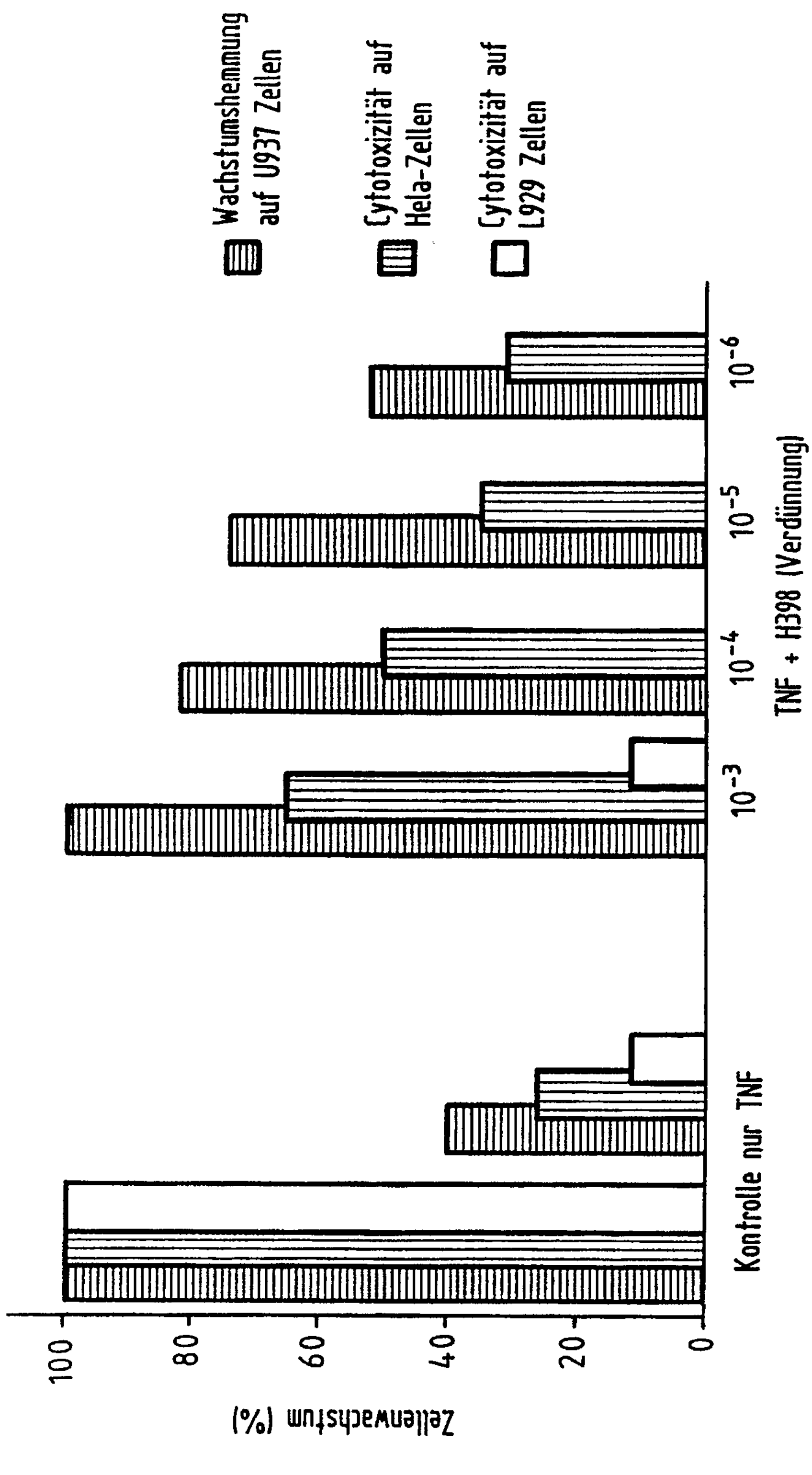

Abb.3

H398 Antikörper antagonisiert die durch TNF-induzierte HLA-Genexpression

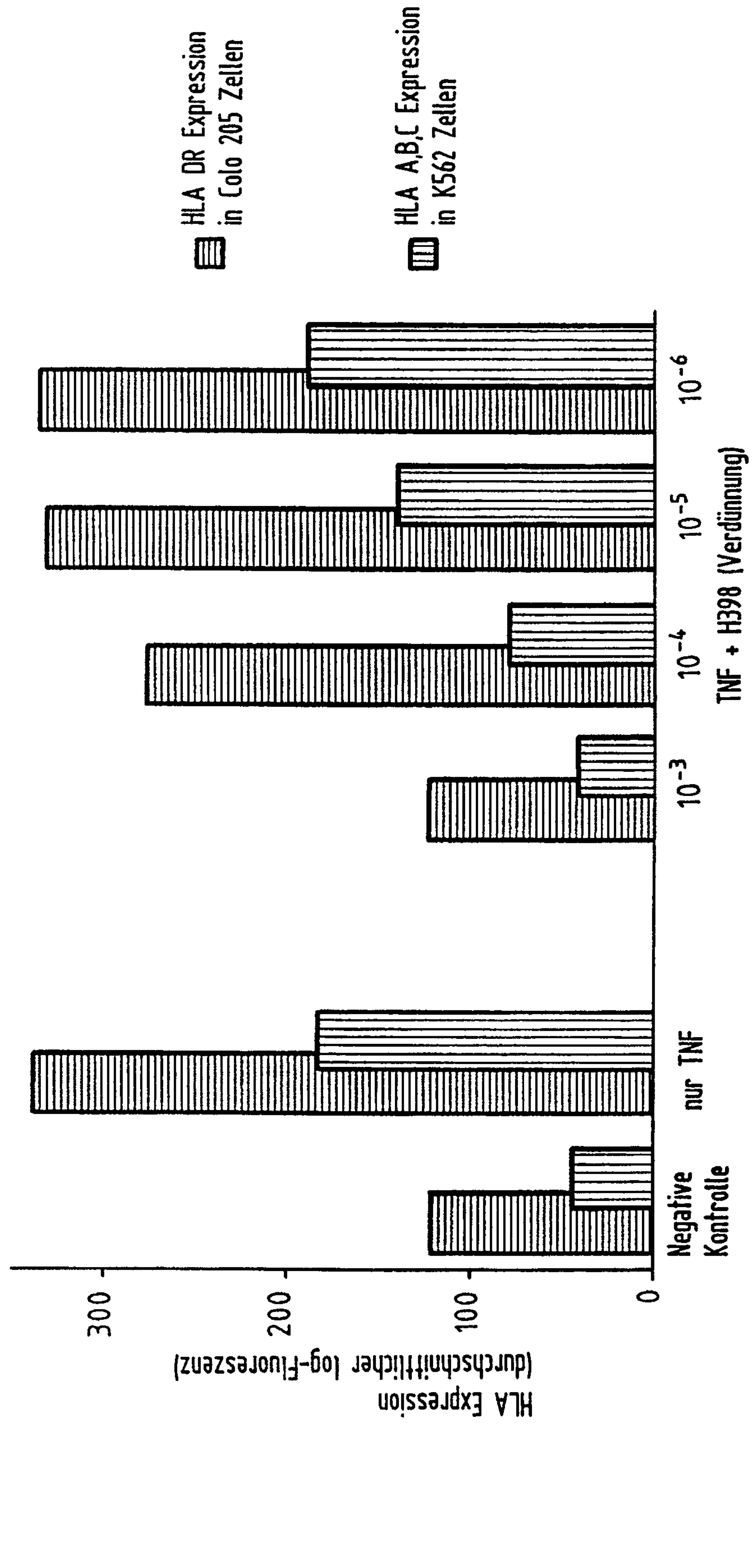

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 91 10 2711

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 334 165 (F. HOFFMANN - LA ROCHE)<br>* Das ganze Dokument *<br>-- | 1-16 | C 12 P 21/08<br>C 12 N 5/20<br>C 07 K 3/28<br>A 61 K 39/395 |
| X | J. EXP. MED., Band 171, Nr. 2, 1. Februar 1990, Seiten 415-426, The Rockefeller University Press, New York, US; T. ESPEVIK et al.: "Characterization of binding and biological effects of monoclonal antibodies against a human tumor necrosis factor receptor"<br>* Zusammenfassung *<br>-- | 1-16 | |
| X | EP-A-0 230 574 (YALE UNIVERSITY)<br>* Seite 7, Zeilen 1-19; Ansprüche *<br>-- ./. | 1-16 | RECHERCHIERTE SACHGEBIETE (Int. Cl.4)<br>C 07 K<br>C 12 P |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-16
Unvollständig recherchierte Patentansprüche:
Nicht recherchierte Patentansprüche: 17
Grund für die Beschränkung der Recherche:

Verfahren zur chirurgischen oder therapeutischen Behandlung des menschlichen oder tierischen Körpers (Siehe Art. 52(4) des Europäischen Patentübereinkommens)

| Recherchenort | Abschlußdatum der Recherche | Prüfer |
|---|---|---|
| DEN HAAG | 30-05-1991 | NOOIJ |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER TEILRECHERCHENBERICHT**

Nummer der Anmeldung

EP 91 10 2711
-2-

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| X | US-A-4 770 995 (B. RUBIN et al.)<br>* Spalte 7, Zeilen 34-56 *<br>-- | 1-9 | |
| X | THE JOURNAL OF BIOLOGICAL CHEMISTRY, Band 265, Nr. 3, 25. Januar 1990, Seiten 1531-1536, The American Society for Biochemistry and Molecular Biology, Inc., Baltimore, US;<br>H. ENGELMANN et al.: "Two tumor necrosis factor-binding proteins purified from human urine"<br>* Seite 1534, rechte Spalte, Zeilen 1-25; Seite 1535, rechte Spalte, Zeilen 4-21; Figur 6 *<br>-- | 1-7 | RECHERCHIERTE SACHGEBIETE (Int. Cl.4) |
| D,X | THE JOURNAL OF BIOLOGICAL CHEMISTRY, Band 264, Nr. 25, 5. September 1989, Seiten 14927-14934, The American Society for Biochemistry and Molecular Biology, Inc., Baltimore, US;<br>H.-P. HOHMANN et al.: "Two different cell types have different major receptors for human tumor necrosis factor (TNFalpha)"<br>* Figur 4 *<br>-- | 1-7 | |
| P,X | J. EXP. MED., Band 172, Nr. 4, 1. Oktober 1990, Seiten 1019-1023, The Rockefeller University Press, New York, US;<br>B. THOMA et al.: "Idnetification of a 60-kD tumor necrosis factor (TNF) receptor as the major signal transducing component in TNF responses"<br>* Der ganze Artikel *<br>---- | 1-16 | |

EPA Form 1505.3 06.78